# EUROPEAN PATENT APPLICATION

(11) **EP 2 796 050 A1**
(43) Date of publication of application: **29.10.2014**
(21) Application number: 13165160.6
(22) Date of filing: 24.04.2013
(51) Int. Cl.: A23C 9/123, A23C 9/133

(54) **Lactic acid bacteria capable of enhancing fruit flavour**

(71) Applicant: Danone GmbH, 85540 Haar (DE)
(72) Inventor: The designation of the inventor has not yet been filed
(74) Representative: Bernardi Tedesco, Céline Michèle Claude

(57) **Abstract**

The present invention relates to a composition comprising lactic acid bacteria and a process for manufacturing fermented dairy products using said composition.

## Description

### Field of the invention

The present invention relates to a composition comprising lactic acid bacteria and a process for manufacturing fermented dairy products using said composition.

### Background of the invention

The food industry uses different bacteria, in the form in particular of ferments, also referred to as starter cultures, in particular lactic acid bacteria, in order to improve the taste and the texture of foods but also to extend the shelf life of these foods. In the case of the dairy industry, lactic acid bacteria are used intensively in order to bring about the acidification of milk (by fermentation) but also in order to texturize the product into which they are incorporated. Among the lactic acid bacteria used in the food industry, there can be mentioned the genera *Streptococcus* and *Lactobacillus.* The lactic acid bacterial species *Streptococcus thermophilus* and *Lactobacillus delbrueckii* ssp *bulgaricus* are used in particular in the formulation of the ferments used for the production of fermented dairy products, typically fermented milks, for example yogurts.

The acidity produced in yogurt depends mainly on the acidifying activity of the yogurt culture (*Streptococcus thermophilus* and *Lactobacillus delbrueckii* ssp. *bulgaricus*) and therefore the amount of lactic acid produced during the milk maturation and also the residual acidity produced during cold storage. The texture is also varying during storage and participates in the final product sensorial properties. The recipe of the yogurt has also an impact on the yogurt sensorial properties by modifying the texture or the aroma perception. For example removing part of the fat in yogurt modifies its properties and for instance maintaining the yogurt texture may be a problem. Various food additives, such as polysaccharides, have been identified to restore the rheological properties and mouthfeel of these reduced-fat products to those of their full-fat counterparts.

Some ferments including *Streptococcus thermophilus* and/or *Lactobacillus delbrueckii ssp. bulgaricus* bacterial strain(s) have been described as providing some fruity flavor notes upon fermenting milk. However the combination of these strains with fruits has not been described and the intensity of such flavors is believed to be much lower than the intensity provided by fruit addition.

In fruit-based products a fruit preparation is added to the yogurt white mass and will give a specific sensorial profile to the yogurt by combining different effects between the yogurt texture and acidity and the fruit preparation taste. Such products usually include further fruit flavors or fruit flavor enhancers to provide a marked fruit taste. On the one hand such flavors or flavor enhancers are not well perceived by some consumers appreciating more natural flavors. Therefore, there is a need for products that reduce or completely abolish the need to use of added flavors (flavoring agents, "e-numbers"). There is also a need for simplified and/or less expensive recipes that allow a reduced amount or free of such flavors or flavor enhancers. There is also a need in products that allow having a further enhanced fruit taste.

Consequently, there is a need for a culture that provides an enhanced fruit flavor when used in a yogurt produced without added flavor. There is a need for products having an appreciated and flavor-intense enough taste that can compete with products having higher amounts of flavor additives.

The inventors have now surprisingly found that selected cultures of lactic acid bacteria are capable of providing more of the body and the mouthfeel of regular full-fat yogurt as can be achieved using ingredients and additives described in the prior art. In addition, the inventor have found that these selected cultures of lactic acid bacteria are capable of enhancing fruit flavour in fruit based dairy products and/or avoiding the need to use of flavours additives in fruit based dairy products.

### Detailed description of the invention

In a first aspect the invention provides a composition comprising one or more bacterial strains selected from the group consisting of strain A, strain B, strain C and strain D and strain E as defined in the MATERIALS and METHODS. Preferred compositions of the invention are the following:
Compositions comprising at least 1 strain from the group consisting of strain A, strain B, strain C and strain D and strain E.
   - Composition 1 comprising strain A
   - Composition 2 comprising strain B
   - Composition 3 comprising strain C
   - Composition 4 comprising strain D
   - Composition 5 comprising strain E

Compositions comprising at least 2 strains from the group consisting of strain A, strain B, strain C and strain D and strain E.
- Composition 6 comprising strain A and strain B
- Composition 7 comprising strain A and strain C
- Composition 8 comprising strain A and strain D
- Composition 9 comprising strain A and strain E
- Composition 10 comprising strain B and strain C
- Composition 11 comprising strain B and strain D
- Composition 12 comprising strain B and strain E
- Composition 13 comprising strain C and strain D
- Composition 14 comprising strain C and strain E
- Composition 15 comprising strain D and strain E

Compositions comprising at least 3 strains from the group consisting of strain A, strain B, strain C and strain D and strain E.
- Composition 16 comprising strain A and strain B and strain C
- Composition 17 comprising strain A and strain B and strain D
- Composition 18 comprising strain A and strain B and strain E
- Composition 19 comprising strain A and strain C and strain D
- Composition 20 comprising strain A and strain C and strain E
- Composition 21 comprising strain A and strain D and strain E
- Composition 22 comprising strain B and strain C and strain D
- Composition 23 comprising strain B and strain C and strain E
- Composition 24 comprising strain B and strain D and strain E
- Composition 25 comprising strain C and strain D and Strain E

Compositions comprising at least 4 strains from the group consisting of strain A, strain B, strain C and strain D and strain E.
- Composition 26 comprising strain A and strain B and strain C and strain D
- Composition 27 comprising strain A and strain B and strain C and strain E
- Composition 28 comprising strain B and strain C and strain D and strain E
- Composition 29 comprising strain A and strain B and strain D and strain E
- Composition 30 comprising strain A and strain C and strain D and strain E

Compositions comprising at least 5 strains from the group consisting of strain A, strain B, strain C and strain D and strain E.
- Composition 31 comprising strain A and Strain B and Strain C and strain D and Strain E

Each of the 31 compositions listed may encompass different embodiments depending on the amount of the strains present in the composition. The individual strains in the compositions may constitute any suitable percentage of the total cfu's (colony forming units) in the compositions. Preferably the compositions of the invention comprise only any of the strains A, B, C, D and E. In those compositions, these strains constitute 100% of the cfu's.

The composition of the invention may however, comprise further other bacterial strains. In those compositions, the total cfu's relates not only to the strains A, B, C, D and E present in the composition but also to the other bacterial strains present in the compositions of the invention.

Strain E in the compositions of the invention (*Lactobacillus delbrueckii* ssp. *bulgaricus* DS71836) comprising 2 or more strains of which at least one strain is strain E, constitutes between 0.1% and 10% of the total cfu's of the composition, preferably between 0,2% and 5%, more preferably between 0,5% and 2%, more preferably between 0,8 and 1,2%, most preferably 1%. Preferably, the *Streptococcus thermophilus* strains A, B, C and D constitute the remaining cfu's of the composition of the invention.

In the compositions comprising one *Streptococcus thermophilus* strain (A or B or C or D) and strain E (compositions 9, 12, 14 and 15), strain E is present as described above, i.e. between 0.1% and 10% of the total cfu's of the composition, preferably between 0,2% and 5%, more preferably between 0,5% and 2%, more preferably between 0,8 and 1,2%, most preferably 1%. In those compositions, the *Streptococcus thermophilus* strain constitutes the remaining cfu's whereby the total cfu's is 100%.

The strains in the compositions comprising two of the *Streptococcus thermophilus* strains A, B, C and D may constitute the individual *Streptococcus thermophilus* strains in any suitable percentage of the total *Streptococcus thermophilus* cfu's in the composition. For instance, in composition 6, strain A may constitute 10% of the total *Streptococcus thermophilus* cfu's in the composition whereby strain B then constitutes 90%. An alternative embodiment of composition 6 may comprise 30% of strain A and 70% of strain B, all relative to the total *Streptococcus thermophilus* cfu's in the composition. A preferred embodiment of composition 6, comprises the *Streptococcus thermophilus* strains in equal amounts, i.e. both constitute 50% of the total *Streptococcus thermophilus* cfu's in the composition.

In the compositions comprising two of the *Streptococcus thermophilus* strains and strain E (compositions 18, 20, 21, 23, 24 and 25), strain E is present as described above, i.e. between 0.1% and 10% of the total cfu's of the composition, preferably between 0,2% and 5%, more preferably between 0,5% and 2%, more preferably between 0,8 and 1,2%, most preferably 1%. In those compositions, the *Streptococcus thermophilus* strains constitute the remaining cfu's whereby the total cfu's is 100%.

The strains in the compositions comprising three of the *Streptococcus thermophilus* strains A, B, C and D may constitute the individual *Streptococcus thermophilus* strains in any suitable percentage of the total *Streptococcus thermophilus* cfu's in the composition. For instance, in composition 16, strain A may constitute 10% of the total *Streptococcus thermophilus* cfu's in the composition, strain B 30% and strain C 60%. An alternative embodiment of composition 6 may comprise 30% of strain A and 50% of strain B and 20% of strain C, all relative to the total *Streptococcus thermophilus* cfu's in the composition. A preferred embodiment of composition 16 comprises the *Streptococcus thermophilus* strains in equal amounts, i.e. both constitute 1/3 of the total *Streptococcus thermophilus* cfu's in the composition. The same may apply to any of the compositions

In the compositions comprising three of the *Streptococcus thermophilus* strains and strain E (compositions 27, 28, 29, and 30), strain E is present as described above, i.e. between 0.1% and 10% of the total cfu's of the composition, preferably between 0,2% and 5%, more preferably between 0,5% and 2%, more preferably between 0,8 and 1,2%, most preferably 1%. In those compositions, the *Streptococcus thermophilus* strains constitute the remaining cfu's whereby the total cfu's is 100%.

The strains in the compositions comprising the four *Streptococcus thermophilus* strains (composition 26) may constitute the individual *Streptococcus thermophilus* strains in any suitable percentage of the total *Streptococcus thermophilus* cfu's in the composition. For instance, in composition 26, strain A may constitute 10% of the total *Streptococcus thermophilus* cfu's in the composition, strain B 30%, strain C 40% and strain D 20%. An alternative embodiment of composition 16 may comprise 30% of strain A and 50% of strain B and 10% of strain C and 10% of strain D, all relative to the total *Streptococcus thermophilus* cfu's in the composition. A preferred embodiment of composition 26 comprises the *Streptococcus thermophilus* strains in equal amounts, i.e. both constitute 1/4 of the total *Streptococcus thermophilus* cfu's in the composition.

In the compositions comprising all four *Streptococcus thermophilus* strains and strain E (composition 31), strain E is present as described above, i.e. between 0.1% and 10% of the total cfu's of the composition, preferably between 0,2% and 5%, more preferably between 0,5% and 2%, more preferably between 0,8 and 1,2%, most preferably 1%. In those compositions, the *Streptococcus thermophilus* strains constitute the remaining cfu's whereby the total cfu's is 100%.

In a preferred embodiment of composition 31 strain E constitutes 1% of the total cfu's and the four *Streptococcus thermophilus* strains constitute 99% of the total cfu's. The individual *Streptococcus thermophilus* strains may constitute in any suitable percentage of the total *Streptococcus thermophilus* cfu's in the composition. The most preferred embodiment of composition 31 comprises 1% of strain E and 24,75% of strain A, 24,75% of B, 24,75% of C and 24,75% of D.

In a second aspect, the invention provides the following bacterial strains of the invention:
- Strain A.: *Streptococcus thermophilus* DS71579 deposited with the Centraalbureau voor Schimmelcultures on 09 April 2013 having deposition number CBS134831.
- Strain B.: *Streptococcus thermophilus* DS71586 deposited with the Centraalbureau voor Schimmelcultures on 09 April 2013 having deposition number CBS134834.
- Strain C.: *Streptococcus thermophilus* DS71584 deposited with the Centraalbureau voor Schimmelcultures on 09 April 2013 having deposition number CBS134832.
- Strain D.: *Streptococcus thermophilus* DS71585 deposited with the Centraalbureau voor Schimmelcultures on 09 April 2013 having deposition number CBS134833.
- Strain E.: *Lactobacillus delbrueckii* ssp. *bulgaricus* DS71836 deposited with the Centraalbureau voor Schimmelcultures on 9 April 2013 having deposition number CBS134835.

In a third aspect, the invention provides a process for the production of a yogurt which has one or more improved properties selected from the group consisting of acidification speed and the maximum gel strength, acidity, mouthfeel and visual aspect of the yogurt obtained, comprising using the composition of the invention, preferably any of the compositions 1-31 or any of the bacterial strains of the invention, i.e. strain A, strain B, strain C, Strain D and strain E. The yogurt may be a full fat yogurt or may be a fat-reduced yogurt.

In a fourth aspect, the invention provides the use of any of the composition as defined by claims 1-8 or bacterial strains as defined by claims 9-13 of the production of a full fat or fat-reduced yogurt.

In a fifth aspect the invention provides a process of making a fermented dairy product comprising the steps of
Step a) providing a milk-based composition,
Step b) inoculating with the composition of the first aspect of the invention, and
Step c) allowing a fermentation
Step d) recovering a white mass.

### Step a) - Milk-based composition

The process involves providing a milk-based composition. Milk-based compositions useful in the process are known by the one skilled in the art of fermented dairy products. Herein a milk-based composition encompasses milk or milk fractions, and compositions obtained by mixing several previously separated milk fractions. Some water or some additives can be added to said milk, milk fractions and mixtures. Herein milk typically refers to animal milk, for example cow milk. Some alternative animal milks can be used, such as sheep milk or goat milk.

The milk-based composition can typically comprise ingredients selected from the group consisting of milk, half skimmed milk, skimmed milk, milk powder, skimmed milk powder, milk concentrate, skim milk concentrate, milk proteins, cream, buttermilk and mixtures thereof. Some water or additives can be mixed therewith. Examples of additives that can be added include sugar, sweeteners different from sugar, fibers, and texture modifiers.

The milk-based composition can typically have a fat content of from 0% to 5%, for example of from 0% to 1% or from 1% to 2% or from 2% to 3% or from 3% to 4% or from 4% to 5%.

The milk-based composition can typically have a fat content of from 2% to 6%, for example of from 2% to 3% or from 3% to 4% or from 4% to 5% or from 5% to 6%.

The ingredients of the milk-based composition and/or the amounts thereof can be selected thereto.

Step a) can comprise sub-steps further to mixing such as heat-treatments, for example pasteurization or sterilization, and/or homogenization. Such steps are known be the one skilled in the art.

Step a) can be performed using conventional equipment such as mixing equipment, heat exchangers, and homogenizers. In a particular embodiment step a) comprises the following steps:
- step a1) mixing ingredients to provide the milk-based composition comprising erythritol,
- step a2) pasteurizing at a temperature of at least 90°C
- step a3) homogenizing and cooling to a temperature of less than 50°C.

Step a) can comprise a homogenization step. This is preferably carried out at step a3). Such operations are well known by the one skilled in the art and can be performed with conventional equipment. The homogenization can be performed at a pressure of at least 25 bars. In a particular embodiment, the homogenization phase is performed at a pressure of at least 100 bars. It is mentioned that the homogenization can be performed in two steps: one at a pressure of 100-200 bars, one at a pressure of 25-50 bars.

Step a) can comprise a heat treatment, such as pasteurization, Ultra High Temperature treatment, or High Temperature treatment. This is preferably carried out at step a2). Such treatments are known by the one skilled in the art, and can be performed with conventional equipments. The heat treatment is typically operated at at least 90°C. Depending on the temperature the treatment time can last typically from 1s to 20 minutes.

Step a) can comprise a step of placing the mixture to a fermentation temperature, typically comprised between 30 and 50°C, preferably of 35°C to 45°C. This is typically done by cooling after a heat treatment. This can be done for example at step a3).

### Step b) - inoculation

Step b) involves inoculating the composition of the first aspect of the invention in the milk-based composition. The operation per se'(inoculation) is known by the one skilled in the art. In addition to the composition of the first aspect of the invention further lactic acid bacteria may be inoculated. Examples of further lactic acid bacteria that can be inoculated include probiotic bacteria. Probiotic bacteria are known by the one skilled in the art.

Examples of probiotic bacteria include some *Bifidobacteria* and *Lactobacilli,* such as *Bifidobacterium brevis, Bifidobacterium animalis, Bifidobacterium animalis lactis, Bifidobacterium infantis, Bifidobacterium longum, Lactobacillus helveticus, Lactobacillus casei, Lactobacillus casei paracasei, Lactobacillus acidophilus, Lactobacillus rhamnosus, Lactobacillus plantarum, Lactobacillus reuteri, Lactobacillus delbrueckii subsp lactis, Lactobacillus delbrueckii subsp delbrueckii, Lactobacillus brevis and Lactobacillus fermentum.*

### Step c) - fermentation

Step c) involves allowing a lactic fermentation. This is typically done at a temperature of higher than 30°C. This step is also referred to as a fermentation step. Step c) leads to a composition referred to as white mass.

Fermentation operations are known by the one skilled in the art. Fermentation can be typically performed at a temperature between 30 and 50°C, preferably from 35°C to 45°C. Fermentation can be stopped by cooling and/or breaking the mixture when a breaking pH is reached. The fermentation time is the time between the inoculation and the breaking and/or cooling. The fermentation time can depend on the lactic acid bacteria, on the amount thereof, and on the temperature, and can for example last from 3 hours to 30 hours, for example from 12 to 22 hours.

During fermentation, the pH of the mixture decreases with production of lactic acid by the bacteria. The pH at the end of the fermentation can be typically of 5 or less than 5, preferably of 3.5 to 4.6. In a preferred embodiment the lactic acid fermentation carried out to a pH of from 3.5 to 5 preferably from 4.5 to 4.9.

After fermentation, step c) can comprise a further step of stirring to obtain a composition having a desired viscosity. Such a step can be performed for example with a smoothing valve, for example at a pressure of at least 1.5 bars, or with a static mixer or with dynamic mixer. This step provides some shear to composition that typically allow a viscosity drop. Such operations are known by the one skilled in the art, and can be operated with conventional appropriate equipment. This step is typically performed at cold temperature, for example at a temperature of form 1°C to 25°C. It is mentioned that this stirring step is not performed in the case of set products, wherein fermentation is performed in packaging.

### Step d) - recovering white mass

In step d) the white mass is recovered. In the case of set products the white mass is recovered directly in the packaging. In the case of stirred or drink products, the white mass is transferred to packing equipment, optionally with mixing and/or association further ingredients and/or components such as fruits, sugar, sweeteners different from sugar, coloring agents, fibers, minerals, vitamins, fat or fat substitutes, for example vegetal fat, preservatives, etc.

In a preferred embodiment, the process of fifth aspect the invention further comprises the step of adding a fruit preparation to the white mass. The amount of fruit preparation added to the white mass may be in the range of 5-35% by weight, based on the total weight of the product (i.e. the fruit containing white mass). In a highly preferred embodiment, the white mass containing the fruit preparation is substantially free of flavor additive(s).

In a sixth aspect of the invention provides a dairy product, obtainable by the process of the fifth aspect of the invention, comprising a white mass being the fermentation product of a milk-based composition and the composition of the first aspect of the invention. Preferred embodiments of the dairy product comprise Strain E and one or more of strain A, B, C and D or Strain A, Strain B, Strain C, Strain D and Strain E. The most preferred embodiment comprises 24.75% of each of Strains A, B, C and D and 1% of Strain E (all based on total CFUs in the composition of the first aspect of the invention.

Preferred dairy products of the sixth aspect of the invention comprise a fruit preparation in addition to the white mass as defined hereinbefore. Herein fruits refer to any fruit forms, including for example full fruits, pieces, purees, concentrates, juices etc.

The fruits can be typically introduced in the product by mixing the white mass with fruits, typically in the form of a fruit preparation. Such introductions and preparation are known by the one skilled in the art. Typically a fruit preparation can be added in an amount of 5-35% by weight with reference to the total amount of product. In a particular embodiment the fruit preparation is free of flavor additive(s).

The fruit preparation typically comprises a stabilizing system, having at least one stabilizer. The stabilizing system can comprise at least two stabilizers. Such stabilizers are known by the one skilled in the art. They typically help in avoiding phase separation of solids, for examples of fruits or fruits extracts and/or in avoiding syneresis. They typically provide some viscosity to the composition, for example a viscosity (Bostwick viscosity at 20°C) of from 1 to 20 cm/min, preferably of from 4 to 12 cm/min. The stabilizing system or the stabilizer can for example be a starch, pectin, a guar, a xanthan, a carrageenan, a locust bean gum, or a mixture thereof. The amount of stabilizing system is typically of from 0.5 to 5% by weight.

The fruit preparation can typically comprise organoleptic modifiers. Such ingredients are known by the one skilled in the art. The organoleptic modifiers can be for example sugars, sweetening agents, coloring agents, cereals and/or cereal extracts.

Examples of sweetening agents are ingredients referred to as High Intensity Sweeteners, such as sucralose, acesulfamK, aspartam, saccharine, rebaudioside A or other steviosides or stevia extracts.

Examples of fruits include for example strawberry, peach, apricot, mango, apple, pear, raspberry, blueberry, passion, cherry, and mixtures or associations thereof, such as peach-passion.
The fruits can be for example provided as:
- frozen fruit cubes, for example 10 mm fruit cubes, for example Individual Quick Frozen fruit cubes, for example strawberry, peach, apricot, mango, apple, pear fruit cubes or mixtures thereof,
- Aseptic fruit cubes, for example 10 mm fruit cubes, for example strawberry, peach, apricot, mango, apple or pear fruit cubes or mixtures thereof,
- fruit purees, for example fruit purees concentrated from 2 to 5 times, preferably 3 times, for example aseptic fruit purees, for example strawberry, peach, apricot, mango, raspberry, blueberry or apple fruit purees or mixtures thereof,
- single aseptic fruit purees, for example strawberry, raspberry, peach, apricot, blueberry or apple single aseptic fruit purees or mixture thereof,
- frozen whole fruits, for example Individual Quick Frozen whole fruits, for example blueberry, raspberry or blackberry frozen whole fruits, or mixtures thereof,
- mixtures thereof.

The ingredients and/or components of fruit preparation and the amounts thereof are typically such that the composition has a brix degree of from 1 to 65 brix.

The fruit preparation can comprise water. It is mentioned that a part of the water can come from ingredients used to prepare the fruit preparation, for example from fruits or fruit extracts or from a phosphoric acid solution. The amount of water in the fruit preparation is preferably of from 10 to 99%, preferably of from 10 to 87%.

The fruit preparation can comprise pH modification agents such as citric acid.

Most preferred are dairy products comprising a fruit preparation in addition to the white mass while being substantially free of flavor additive(s). In the present application a flavor additive refers to a compound or composition, of matter, different from a fruit, added to the product or an intermediate thereof to provide a flavor taste modification. Flavor additive encompass aromas and are known by the one skilled in the art. They are typically labeled on dairy fermented products. Flavors are for example listed on European regulation EU 872/2012. Examples of flavors include some acids, esters, ketones, terpens, alcohols, benzoic cycles, fatty acids. Examples of common flavors, typically used in strawberry flavoring are methyl-butyrate, 3-6 hexenol, furaneol, caproic acid, decalactone.

In the present application a product "substantially free of flavor additive" refers to a product that does not comprise significant amounts of flavor additive. The amount might depend on the very flavor additive considered. The amount is typically of lower than 1%, preferably lower than 0.1%, preferably lower than 0.01%, preferably lower than 0.001%, preferably lower than 1 ppm, preferably lower than 0.1 ppm, preferably lower than 0.01 ppm, preferably none. By "substantially free of flavor additive" it is preferably meant that no flavor additive is added and/or that the presence of such compounds is not labeled.

The product is typically to be used as a food product. It is typically used by oral administration. One can typically eat or drink the composition by processing it from a container to the mouth, optionally using a spoon, a glass, or a straw. The container is preferably a cup.

The product is typically conditioned by filling in a container, such as a cup or a bottle, and then sealing, to obtain a finished product. Sealing can be performed for example with a cap or with a lid. The container can be for example a container of 50 ml (or 50 g) to 1 L (or 1 kg), for example a container of 50 ml (or 50 g) to 80 ml (or 80 g), or 80 ml (or 80 g) to 100 ml (or 100g), or 100 ml (or 100 g) to 125 ml (or 125 g), or 125 ml (or 125 g) to 150 ml (or 150 g), or 150 ml (or 150 g) to 200 ml (or 200 g), or 250 ml (or 250 g) to 300 ml (or 300 g), or 300 ml (or 300 g) to 500 ml (or 500 g), or 500 ml (or 500 g) to 750 ml (or 750 g(, or 750 ml (or 750 g) to to 1 L (or 1 kg).

The product can be stored, transported and/or distributed at a chilled temperature of 0°C to 10°C, preferably of 4°C to 10°C.

### MATERIALS AND METHODS

### Bacterial strains and blends of strains (culture).

**Table 1. Bacterial strains**

| **Strain** | **CBS number** | **Strain** |
|---|---|---|
| A | CBS134831 | *Streptococcus thermophilus* DS71579 |
| B | CBS134834 | *Streptococcus thermophilus* DS71586 |
| C | CBS134832 | *Streptococcus thermophilus* DS71584 |
| D | CBS134833 | *Streptococcus thermophilus* DS71585 |
| E | CBS134835 | *Lactobacillus delbrueckii ssp. bulgaricus* DS71836 |

All strains A-E were deposited on 9 April 2013 at the Centraalbureau voor Schimmelcultures (Fungal Biodiversity Centre), Uppsalalaan 8, 3584 CT Utrecht, The Netherlands under the provisions of the Budapest Treaty.

### Blends

The following blends were used in the Examples. The percentages relate to the cfu's (colony forming units) - see Table 2.

**Table 2**

| Blend | Strain A | Strain B | Strain C | Strain D | Strain E |
|---|---|---|---|---|---|
| 1 | 24,75% | 24,75% | 24,75% | 24,75% | 1% |
| 2 | 24,75% | 24,75% | 16,5% | 33,0% | 1% |
| 3 | 25,20% | 25,20% | 16,5% | 33,0% | 0.1% |

The reference cultures are commercially available culture comprising at least one *Streptococcus thermophilus* bacterial strain, and at least one *Lactobacillus delbrueckii* ssp. *Bulgaricus.* The reference cultures do not contain any of strains A-E.

### Yogurt preparation (both regular and reduced fat) - Example 1 and 2

The fermented milk used is obtained by supplementing pasteurized skimmed milk (Isigny) with skimmed milk powder and cream (35% fat). The final recipe is described in the different examples.

The milk mixture is pasteurized at 95°C during 6 minutes at 180 bars. The pasteurized milk mix is then inoculated with the culture to be tested at a rate of 0,02% (w/w) and incubated at 38°C in a water bath until a pH of 4,60 is reached. The monitoring of the pH is continuously recorded. Fermented milk then obtained is stirred, smoothen and cooled down at 22°C before filling in the cups. The yogurt cups are then stored at 4°C.

### Texture analysis of yogurt - Example 1 and 2

During storage, the rheological properties of the samples were measured using a rheometer (Haake VT550 ThermoFischer Scientific) equipped with a Haake vane, Immersion Sensor system FL measuring system and a coaxial cylinder with cup, Sensor System DIN 53019 measuring system.
The yogurt resistance was measured, caused by a deformation applied by a vane, during 120s, at a constant shear rate (0.04000 1/s). The value of the maximal yogurt strength (µNM) is correlated to the sensorial evaluation of the thickness in cup.
The yogurt viscosity was also measured with shear rates varying from 0.27 to 300 s ⁻¹. Shear rates were increased and then decreased and the upward and downward curves of shear stress (Pascal, Pa) and apparent viscosity (Pa*s) were recorded. For further analysis, shear stress at 300 s⁻¹ was chosen and gave a good correlation to the sensorial evaluation of mouth thickness.

### Sensorial analysis of yogurts - Example 1 and 2

The sensory panel consisted of 8 members who had a specific training in sensory evaluation of yogurts. Products were presented in 3-digit coded, white plastic isothermal cups stored at 4oC. The samples were at approximately 10 °C when they were tested. Panelists were provided with mineral water for palate cleaning between samples. The sessions were carried out in a temperature controlled room at 20°C under white lighting in individual booths. Data acquisition was assisted by FIZZ Sensory Analysis Software. Both monadic and hedonic scales are being used to rate the flavor and the texture attributes of products. The attributes were evaluated in the following order: visual texture with a spoon, texture-in-mouth. taste, aroma.

### Visual aspect of the yogurt - Example 1 and 2

The aspect of the yogurt is assessed visually by taking a spoonful of yogurt and evaluating if the product is grainy / sandy or smooth.

### White mass

The white mass is a fermented composition, typically a fermented milk product, such as yogurt, kefir; cheese etc.... Fermented milk products are known by the one skilled in the art. Such products are made from a milk-based composition and have undergone a fermentation step. The fermentation is typically done by microorganisms comprising lactic acid bacteria and optionally yeasts, and leads to the production of fermentation products, for example lactic acid, and/or to the multiplication of the microorganisms. The designation "fermented milk" can depend on local legislation, but is typically given to a dairy product prepared from skimmed or full fat milk, or concentrated or powdered milk, having undergone a heat treatment at least equivalent to a pasteurization treatment, and inoculated with lactic acid producing microorganisms. The white mass and the product are preferably a yogurt.

In the present invention the microorganisms comprise the strains of the starter culture. It is mentioned that further microorganisms, preferably further lactic acid bacteria, can be introduced. Examples of further lactic acid bacteria include further *Lactobacilli (Lactobacillus acidophilus, Lb. casei, Lb. plantarum, Lb. reuteri, Lb. johnsonii), further Streptococci (Streptococcus thermophilus), Bifidobacteria (Bifidobacterium bifidum, B. longum, B. breve, B. animalis) and*/*or Lactococci (Lactococcus lactis).*

The white mass can be a set product, wherein fermentation occurs in the packaging or a stirred or drink product, wherein fermentation occurs in a tank, and is then stirred to lower the viscosity prior to pack.

### Fruit preparation

The fruit preparation used in the Examples has the following composition

| | |
|---|---|
| Strawberry IQF cubes | 55.9% |
| Strawberry puree 3 fold concentrate | 4.1% |
| Sucrose | 12.0% |
| Water | 23.2% |
| Starch | 3% |
| Locus bean gum | 0.5% |
| Beet root juice concentrate | 1.3% |

The pH was adjusted to 3.4. Brix=21°. This fruit preparation is free of additional flavors.

### Skim milk powder

Skim Milk powder 1: Nutrilac 7700, Arla
Skim Milk powder 2: Nutrilac 45-85, Arla

### FIGURE LEGENDS

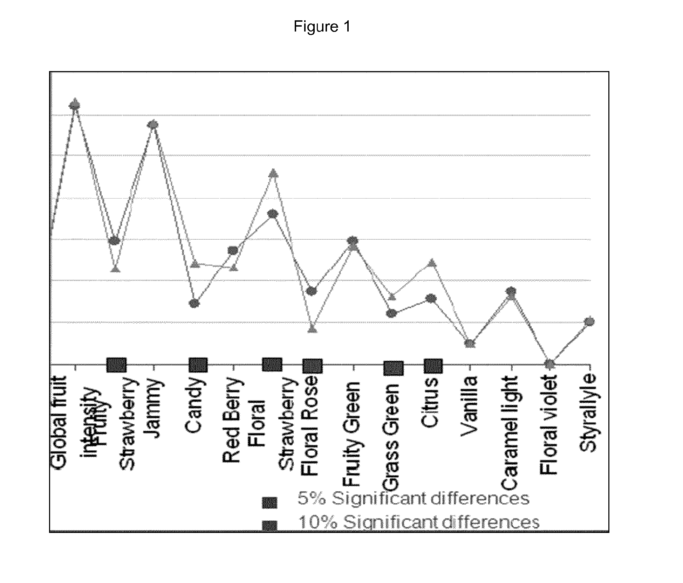

Figure 1
   The strawberry aroma sensory profiles for Example 7.1 and 7.4 (comparative) are represented in Figure 1, where the ordinates represents the average marking (from 1 to 7) and where the circle symbols correspond to comparative example 7.4 and the triangles correspond to Example 7.1.

### EXAMPLES

### Example 1

### Effect of lactic acid bacterial strains on various properties of an unflavored, full fat yogurt.

### Recipe of the yogurts.

For the preparation of the full fat yogurt, the following milk mixture was prepared (see also Materials & Methods).

**Table 3.**

| **Ingredient** | **Weight %** |
|---|---|
| Skimmed Milk | 76.0 |
| Skimmed Milk Powder | 0.8 |
| Cream (35% fat) | 15.7 |
| Sucrose | 7.5 |

The milk mixture containing the above ingredients is then is preheated to 65°C and homogenized at 180 bars and pasteurized to 95°C for 6 minutes to inactivate any organisms or enzymes present in the milk mix. The milk mixture is then cooled down to about 38°C and inoculated with the culture as indicated below at 0.02% (w/w).
The milk mixture containing the culture is then kept at 38°C in a water bath until the pH reaches a value of 4,65 - 4,60. Following this step of fermentation, the yogurt is stirred, cooled at 22°C, filled in cups and stored at 4°C to stop the fermentation but not inactivate or kill the culture.

**Table 4**

| Culture: | Reference | Blend 1 | Blend 2 | Blend 3 |
|---|---|---|---|---|
| Time to reach pH=4,6 (min) | 526 | 318 | 310 | 302 |
| Viscosity at 300 s⁻¹ (Pa) * | 0.254 | 0.251 | 0.260 | 0.262 |
| Maximum Gel strength (µNm) * | 1837 | 1794 | 1837 | 1858 |
| pH * | 4.46 | 4.12 | 4.11 | 4.13 |
| Acidity * | 81 | 97 | 98 | 95 |
| Mouthfeel * | - | ++ | ++ | ++ |
| Aspect * | Smooth | Smooth | Grainy | Grainy |

| | | | | |
|---|---|---|---|---|
| * all measured in the yogurt after 14 days of cold storage of the yogurt | | | | |

### Example 2

### Effect of lactic acid bacterial strains on various properties of an unflavored full fat and reduced fat yogurt.

For the preparation of the full fat and reduced fat yogurt, the following milk mixtures was prepared (see also Materials & Methods).

**Table 5**

| Ingredient | Reduced fat recipe | Full fat recipe |
|---|---|---|
| | Weight % | Weight % |
| Skimmed Milk | 76.0 | 71.1 |
| Skimmed Milk Powder | 0.8 | 0.0 |
| Cream (35% fat) | 15.7 | 21.4 |
| Sugar | 7.5 | 7.5 |

The two recipes differ in fat content. The full fat recipe has 7,5% fat and the reduced fat recipe has 5,5% fat.

**Table 6**

| Culture | Reference | Reference | Blend 1 |
|---|---|---|---|
| Fat | Full | Reduced | Reduced |
| Time to reach pH=4,6 (min) | 342 | 370 | 314 |
| Viscosity at 300 s⁻¹ (Pa) * | 0.263 | 0.244 | 0.259 |
| Maximum Gel strength (µNm) * | 1860 | 1330 | 1245 |
| pH * | 4.40 | 4.43 | 4.11 |
| Acidity * | 72 | 77 | 94 |
| Mouthfeel * | ++ | + | ++ |

| | | | |
|---|---|---|---|
| * all measured in the yogurt after 14 days of cold storage of the yogurt | | | |

### Example 3 - White Mass preparations

A milk-based composition is prepared by mixing 79.79 parts of skim milk, 9.01 parts of cream, 2.44 parts of water, 1.40 parts of skim milk powder 1, 0.25 parts of skim milk powder 2, and 7.00 parts of sugar. The milk-based composition has the following:
- Fat: 3.80%
- Proteins: 4.30%

The milk-based composition is pre-heated to 75°C, then pasteurized at 95°C for 6 minutes, then homogenized at 250 bars then 40 bars, then cooled to 40°C. 0.02 parts of the culture is inoculated, then a fermentation is allowed at 40°C to a pH break of 4.7. The obtained mass is sheared with Ytron Z250 (tangential speed 3.87 m/s) then cooled to 20°C, to obtain a yogurt white mass.

**Table 7**

| Example | Example 3.1 | Example 3.2 | Example 3.3 |
|---|---|---|---|
| Culture used | Blend 1 | Reference 2 | Reference 3 |

### Example 4 - Yogurt with strawberry

80% of the white masses of example 3 are mixed with 20% of the fruit preparation (see Material and Methods). The product obtained is then packaged in a 100 g container and stored at 4°C. The products are tasted after 15 days storage, by a panel of 3 trained people. Observations about taste are reported below:

**Table 8**

| Example | | 4.2 | 4.3 |
|---|---|---|---|
| Culture in white mass | Blend 1 | Reference 2 | Reference 3 |
| White Mass (wt%) | Example 3.1 (80%) | Example 3.2 (80%) | Example 3.3 (80%) |
| Fruit preparation (wt%) | 20% | 20% | 20% |
| Comments | Sour, fruits taste supported and enhanced | Mild, flat taste, not fruity | Very mild not fruity |

Yogurt of Example 4.1 is further tasted with comparison to a benchmark strawberry yogurt comprising flavors (Ehrmann Almighurt) in a mondic 120 people test, after 15 days storage. Both products were evaluated as having a similar overall acceptance, fruit taste intensity is evaluated as being just rights for both products.

### Example 5 - White Mass preparations

A milk-based composition is prepared by mixing 70.63 parts of skim milk, 12.10 parts of cream, 10.25 parts of skim milk concentrate (36%) and 7.00 parts of sugar. The milk-based composition has the following:
- Fat: 3.68%
- Proteins: 4.3%

The milk-based composition is pre-heated to 75°C, then pasteurized at 95°C for 6 minutes, then homogenized at 250 bars then 40 bars, then cooled to 40°C. 0.02 parts of the culture is inoculated and then a fermentation is allowed at 40°C to a pH break of 4.7. The obtained mass is sheared with Ytron Z250 (tangential speed 3.87 m/s) then cooled to 20°C, to obtain a yogurt white mass.

**Table 9**

| Example | Example 5.1 | Example 5.2 | Example 5.4 |
|---|---|---|---|
| Culture used | Blend 1 | Reference 2 | Reference 4 |

### Example 6 - Yogurt with strawberry

80% of the white masses of example 5 are mixed with 20% of the fruit preparation (see Materials and Methods). The product obtained is then packaged in a 100 g container and stored at 4°C. The products are tasted after 15 days storage, by a panel of 3 trained people. The following features are evaluated and reported below:
- Overall acceptance score between 0 and 10 (10 being the best score),
- Fruit intensity score between 0 and 5 (5 being the best score)
- comments

**Table 10**

| Example | 6.1 | 6.2 | 6.3 |
|---|---|---|---|
| Culture in white mass | Blend 1 | Reference 2 | Reference 4 |
| White Mass (wt%) | Example 5.1 (80%) | Example 5.2 (80%) | Example 5.4 (80%) |
| Fruit preparation (wt) | 20% | 20% | 20% |
| Overall acceptance | 7.3 | | 6.2 |
| Fruit intensity | 2.9 | | 2.6 |
| Comments | Fruity, fresh jammy | Flat, not fruits | Cheesy, Less fruity |

### Example 7 - Yogurt with strawberry and Sensory profiles

78% of the white masses of example 5 are mixed with 22% of a fruit preparation identical to the fruit preparation used in previous examples, except that it is slightly more concentrated (concentration factor of 1.1). The product obtained is then packaged in a 100 g container and stored at 4°C.

**Table 11**

| Example | 7.1 | 7.4 | 7.5 - Benchmark |
|---|---|---|---|
| Culture in white mass | Blend 1 | Reference 4 | Strawberry yogurt |
| White Mass (wt%) | Example 3.1 (78%) | Example 3.4 (78%) | comprising flavors |
| Fruit preparation (wt%) | 22% | 22% | (Ehrmann Almighurt) |

The products of Examples 7.1, 7.4 (comparative) and 7.5 (comparative) are evaluated by a panel 12 expert judges, especially trained in evaluating spoonable plain or strawberry yogurts, according to a pure monadic, 2 repetition mode.

The strawberry aroma sensory profiles for Example 7.1 and 7.4 (comparative) are represented in Figure 1, where the ordinates represents the average marking (from 1 to 7), and where the circle symbols correspond to comparative example 7.4 and the triangles correspond to Example 7.1.

The results are further outlined below for Example 7.1 and comparative example 7.4:
- Global fruit: 7.1 equivalent to 7.4 (comparative)
- Fruit intensity: 7.1 slightly lower than 7.4 (comparative)
- Strawberry Jammy: 7.1 equivalent to 7.4 (comparative)
- Candy 7.1 higher than 7.4 (comparative)
- Red Berry 7.1 equivalent to 7.4 (comparative)
- Floral Strawberry 7.1 higher than 7.4 (comparative)
- Floral Rose 7.1 lower than 7.4 (comparative)
- Grass Green 7.1 higher than 7.4 (comparative)
- Citrus 7.1 higher than 7.4 (comparative)
- Vanilla 7.1 equivalent to 7.4 (comparative)
- Caramel light 7.1 equivalent to 7.4 (comparative)
- Floral violet 7.1 equivalent to 7.4 (comparative)
- Styrralyle 7.1 equivalent to 7.4 (comparative)
The results are further outlined below for Example 7.1 and comparative example 7.5:
- Global fruit: 7.1 equivalent to 7.5 (comparative)
- Fruit intensity: 7.1 higher than 7.5 (comparative)
- Strawberry Jammy: 7.1 higher than 7.5 (comparative)
- Candy 7.1 higher than 7.5 (comparative)
- Red Berry 7.1 equivalent to 7.5 (comparative)
- Floral Strawberry 7.1 equivalent to 7.5 (comparative)
- Floral Rose 7.1 equivalent to 7.5 (comparative)
- Grass Green 7.1 equivalent to 7.5 (comparative)
- Citrus 7.1 higher than 7.5 (comparative)
- Vanilla 7.1 lower than 7.5 (comparative)
- Caramel light 7.1 equivalent to 7.5 (comparative)
- Styrralyle 7.1 lower than 7.5 (comparative)

## Claims

1. A composition comprising one or more bacterial strains selected from the group consisting of *Streptococcus thermophilus* DS71579, *Streptococcus thermophilus* DS71586, *Streptococcus thermophilus* DS71584, *Streptococcus thermophilus* DS71585 and *Lactobacillus delbrueckii ssp. bulgaricus* DS71836.

2. A composition comprising *Streptococcus thermophilus* DS71579.

3. A composition comprising *Streptococcus thermophilus* DS71586.

4. A composition comprising *Streptococcus thermophilus* DS71584.

5. A composition comprising *Streptococcus thermophilus* DS71585.

6. A composition comprising *Lactobacillus delbrueckii ssp. bulgaricus* DS71836.

7. A composition comprising *Streptococcus thermophilus* DS71579 and *Streptococcus thermophilus* DS71586 and *Streptococcus thermophilus* DS71584 and *Streptococcus thermophilus* DS71585 and *Lactobacillus delbrueckii ssp. Bulgaricus* LB55.

8. A composition according to claim 6 wherein all four *Streptococcus* strains constitute 24.5% and *Lactobacillus delbrueckii ssp. bulgaricus* DS71836 constitutes 1% of the total cfu's in the composition.

9. *Streptococcus thermophilus* DS71579 deposited with the Centraalbureau voor Schimmelcultures on 9 April 2013 having deposition number CBS134831.

10. *Streptococcus thermophilus* DS71586 deposited with the Centraalbureau voor Schimmelcultures on 9 April 2013 having deposition number CBS134834.

11. *Streptococcus thermophilus* DS71584 deposited with the Centraalbureau voor Schimmelcultures on 9 April 2013 having deposition number CBS134832.

12. *Streptococcus thermophilus* DS71585 deposited with the Centraalbureau voor Schimmelcultures on 9 April 2013 having deposition number CBS134833.

13. *Lactobacillus delbrueckii ssp. bulgaricus* DS71836 deposited with the Centraalbureau voor Schimmelcultures on 9 April 2013 having deposition number CBS134835.

14. A process for the production of a yogurt which has one or more improved properties selected from the group consisting of acidification speed and the maximum gel strength, acidity, mouthfeel and visual aspect of the yogurt obtained, comprising using the composition as defined by anyone of claims 1-8 or any of the bacterial strains as defined by claims 9-13.

15. The process according to claim 14 wherein the yogurt is a full fat yogurt.

16. The process according to claim 14 wherein the yogurt is a fat-reduced yogurt.

17. Use of any of the composition as defined by claims 1-8 or bacterial strains as defined by claims 9-13 of the production of a full fat or fat-reduced yogurt.
